# EUROPÄISCHE PATENTSCHRIFT

(11) **EP 2 189 122 B1**
(45) Veröffentlichungstag und Bekanntmachung des Hinweises auf die Patenterteilung: **21.06.2017**
(21) Anmeldenummer: 09012141.9
(22) Anmeldetag: 24.09.2009
(51) Int. Cl.: A61B 17/15

(54) **System zum Ausrichten eines Schneidblocks an einem Knochen**
System for orientating a cutting block on a bone
Système pour l'orientation d'un bloc de coupe sur un os

(30) Priorität: 24.09.2008 IT PO20080011
(43) Veröffentlichungstag der Anmeldung: 26.05.2010
(73) Patentinhaber: WALDEMAR LINK GmbH & Co. KG, 22339 Hamburg (DE)
(72) Erfinder: Baldini, Andrea, 59100 Prato-Po (IT); Festa, Gaetano, 50066 Regello Loc. Tosi-Fi (IT)
(74) Vertreter: Glawe, Delfs, Moll

(56) Entgegenhaltungen:
- WO-A-01/66021
- US-A- 4 703 751

## Beschreibung

Im Namen von ANDREA BALDINI, italienischer Staatsbürger, wohnhaft in der Via San Giorgio 12, Prato (PO), Italien, und GAETANO FESTA, italienischer Staatsbürger, wohnhaft in der Via IV Novembre 77, Reggello (FI), Italien.

### ZUSAMMENFASSUNG

Die Erfindung ermöglicht dank der wirksamen Steuerung der Sagittalebene (Beugung/Streckung) die Ausführung des distalen Femurschnitts unter Reproduktion von Berechnungen auf der Grundlage vorbereitender Radiographie, ohne Bezugnahme auf den intramedullären Femurkanal.

### BESCHREIBUNG

Die Erfindung wurde mit dem Ziel entwickelt, den bis heute bestehenden Nachteil auszuräumen und den distalen Femurschnitt ohne Verletzung des Femurkanals auszuführen, wobei die gleiche Präzision wie mit dem derzeit zur Verfügung stehenden intramedullären Instrumentarium gewährleistet wird.

Zur Ausführung des distalen Femurschnitts sieht das gewöhnlich eingesetzte Verfahren die Verwendung von Instrumenten vor, die in den Femurschaftkanal eindringen, nachdem dieser mit einem Bohrer über etwa zwanzig Zentimeter verletzt wurde. Das Hauptinstrument weist einen langen Stab mit einem Durchmesser von etwa 8 - 10 mm auf, der über eine Länge von etwa 20 Zentimetern in den Femur eindringt. Dies stellt sicher, dass die in Folge außen angelegten Instrumente eine sichere Referenz zum Femurschaft aufweisen.

Dieses klassische "intramedulläre" System bietet den Vorteil, einfach anwendbar zu sein und nur selten zu groben Positionierungsfehlern zu führen, weist aber den Nachteil auf, einen zusätzlichen invasiven Vorgang für die anatomischen Strukturen (den intramedullären Femurkanal) mit sich zu bringen, was das Risiko der Verursachung einer Fettembolie birgt, und ist außerdem in 15% der Fälle in der Frontalebene und in 20 - 25% der Fälle in der Sagittalebene nicht von absoluter Präzision (0 +/- 2°). Ein System mit den Merkmalen der Präambel des Anspruchs 1 ist aus der Patentanmeldung US 4,703,751 bekannt. Außerdem ist in Fällen von Deformitäten des Femurschafts, wie etwa aufgrund von Brüchen oder Fehlbildungen, oder in Gegenwart einer Hüftprothese oder Nägel/Platten an derselben Seite die Verwendung von intramedullären Instrumenten mit langem Stab unmöglich, und derzeit werden in diesen Fällen in Ermangelung anderer Vorrichtungen ähnliche Instrumente mit kurzem Stab von nur höchst approximativer Präzision verwendet.

Die einzige derzeit angewendete Alternative zu diesem System ist die Verwendung von Systemen der computergestützten "Navigation" während der Operation, mit deren Hilfe der distale Femurschnitt ohne intramedulläre Instrumente auf genaue Weise ausgeführt werden kann. Der Nachteil dieser Systeme liegt hauptsächlich in den Verwendungskosten und in der Verlangsamung der einzelnen chirurgischen Phasen aufgrund der kontinuierlichen Erfassung und Verifizierung der Daten.

Erstrebenswert wäre ein System, das mindestens die gleiche Präzision wie das intramedulläre Instrumentarium ohne Verletzung des Femurkanals gewährleistet.

Die Erfindung ermöglicht die Reproduktion der exakten Bedingungen, die sich aus der vorbereitenden radiographischen Berechnung ergeben, im Operationssaal und das Entfernen des exakten Umfangs an Knochen an der inneren Flanke (Condylus medialis) und der äußeren Flanke (Condylus lateralis) des Femurs, wobei die Reproduktion aller "Offset"-Bedingungen (Bedingungen des unterschiedlichen Abstands in Bezug auf eine Ebene), die die distalen Femurknorren Condylus medialis und Condylus lateralis bei den verschiedenen existierenden Typen arthrotischer Deformität aufweisen können, ermöglicht ist.

Die Recherche in der wissenschaftlichen Literatur hat gezeigt, dass kein Instrumentarium für die Knieprothese mit diesen Merkmalen existiert und auch in der Vergangenheit nie ein solches vorgeschlagen worden ist.

Die Anmelder gehen daher davon aus, dass die Erfindung von allen Akteuren des Sektors besonders geschätzt werden wird, da bis heute kein Instrument zur Verfügung stand, das den wichtigen distalen Femurschnitt unter Reproduktion von Berechnungen auf der Grundlage vorbereitender Radiographie, ohne Bezugnahme auf den intramedullären Femurkanal, ausführt.

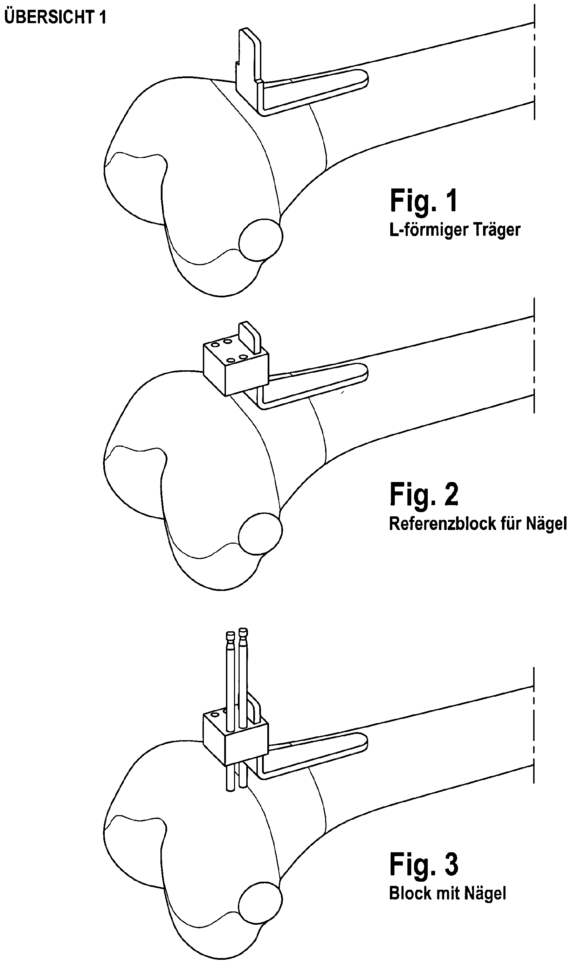

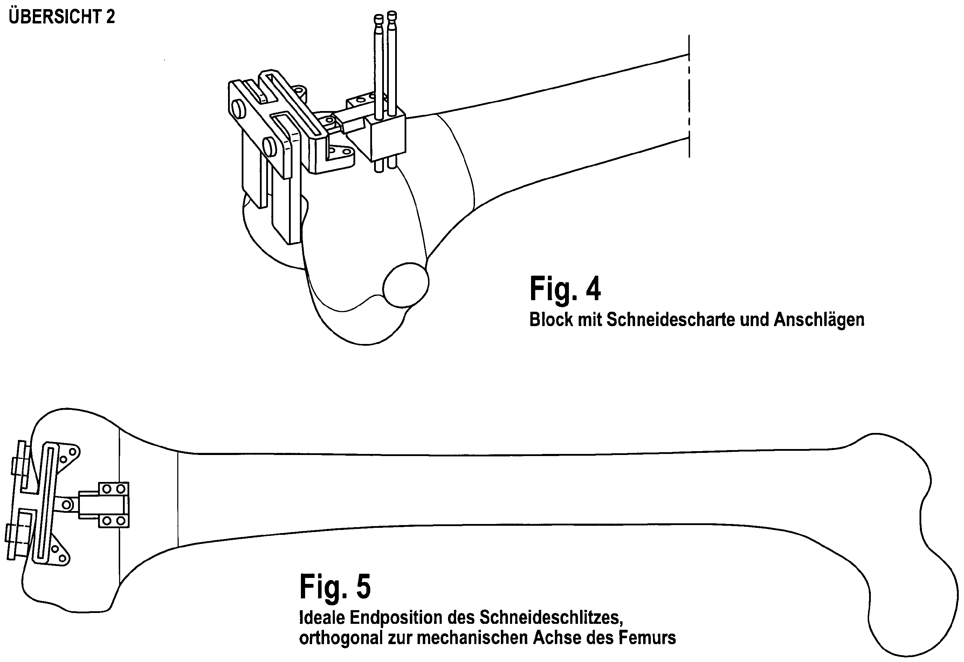

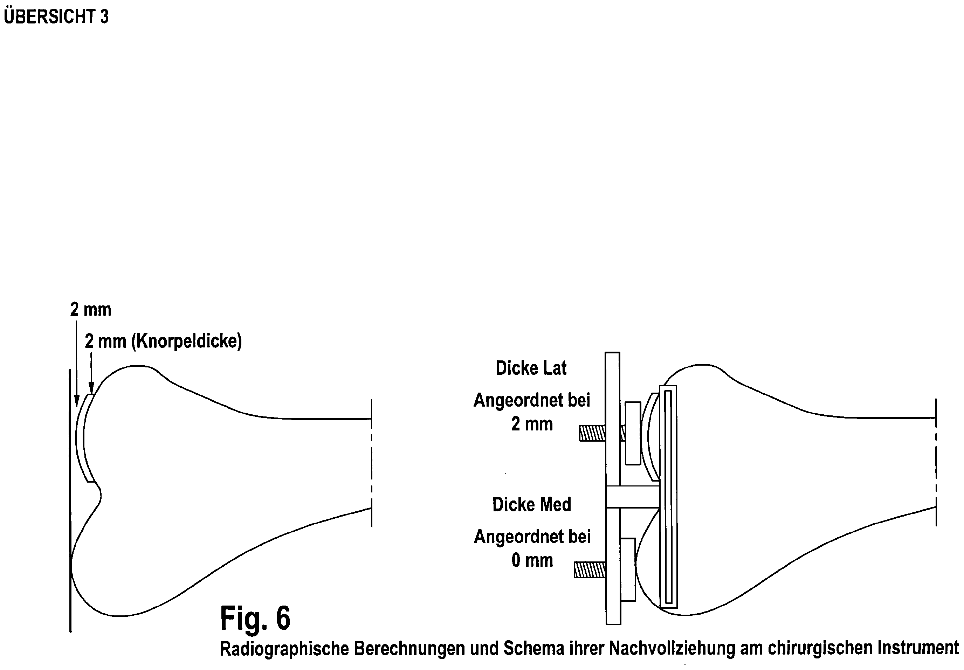

Im Wesentlichen umfasst die Erfindung Folgendes:
1) Einen Träger von der Form eines "L" mit einem Winkel von 90° (Fig. 1, Übersicht 1), der dazu verwendet wird, die Dimension der Beugung/Streckung des gesamten Apparats zu steuern, und dann das Ausführen eines distalen Femurschnitts senkrecht zur sagittalen distalen Femurachse ermöglicht. Wird eingeführt unter dem Stratum synoviale an der medialen Kortikalis des Femurs in unmittelbarer Nähe zur Trochlea des Femurs.
2) Einen Metallblock mit vier Bohrungen an den medialen und lateralen Rändern (Fig. 2, Übersicht 1) und einem Fenster, in dem der kurze Arm des L-förmigen Trägers aufgenommen wird. Zweck des Instruments ist das Ermöglichen des Einführens zweier paralleler Nägel am distalen Femur, die die orthogonale Position zur sagittalen distalen Femurachse halten. Dieses Instrument ist notwendig und darf nicht einstückig mit dem L-förmigen Instrument ausgebildet sein, um Schwierigkeiten beim Herausziehen der Instrumente zu vermeiden, wenn die beiden orthogonalen Nägel erst eingeführt sind. Alternativ zu einfachen Nägeln, die mithilfe eines Hammers einzuführen sind, können Gewindezapfen verwendet werden, die durch Einschrauben einzuführen sind.
3) Einen weiteren Metallblock mit vier Bohrungen an den medialen und lateralen Rändern (Fig. 3, Übersicht 1), die an derselben geometrischen Position wie beim vorherigen Block angeordnet sind. Weist an seiner Mitte eine "Schwalbenschwanz"-Führung auf, um einen Spiegelschlitten des Endinstruments aufzunehmen, das den Schneideschlitz aufweist. Sein Nutzen besteht darin, in die zuvor eingeführten Nägel unter größtmöglicher Einschränkung des Raumbedarfs einzugreifen und das Endinstrument unter Steuerung dessen orthogonaler Position zur sagittalen distalen Femurachse aufzunehmen.
4) Einen Block mit Schneideschlitz (Fig. 4, Übersicht 2), verbunden mit der Plattform mit Aufnahmen für Distanzstücke aus Metall, die zu den distalen Condylen des Femurs in Kontakt geraten, umfassend eine Gelenkkupplung mit "Schwalbenschwanz"-Verbindung, die in den vorherigen Block eingreift. Der Abstand zwischen Plattform und Schneideschlitz beträgt 15 mm. Dieses Instrument steuert durch die Verwendung verschiedener Distanzstücke aus Metall in den beiden Aufnahmen den Varus-/Valgus-Resektionswinkel des Femurschnitts und die Resektionstiefe (entfernte Knochenmenge), wobei die radiographischen Messungen nachvollzogen werden (Fig. 5, Übersicht 2 und Fig. 6, Übersicht 3).

Dieses Instrument kann auch anstelle der verschiedenen Distanzstücke aus Metall (von 3, 5, 7 mm) mit einem System ausgestattet sein, das das Gleiten einer Millimeter-"Kolben"-Führung auf beiden Flanken, medial und lateral, sicherstellt.

Der Gesamtumfang an Knochen, der entfernt wird, wird von der Höhe der Fixierung des Körpers, der die mediale und die laterale Plattform enthält, in Bezug auf den Schneideschlitz bestimmt. Die möglichen Einstellungen ermöglichen das Entfernen von vorbestimmten Knochendicken vom prominenteren Condylus.

## Patentansprüche

1. System zum Ausrichten eines Schneidblocks mit einem Schneidschlitz an einem Knochen, insbesondere an dem distalen Femurende, umfassend einen L-förmigen Träger,
**dadurch gekennzeichnet, dass**
der Träger zur Auflage auf dem Knochen, insbesondere auf dem distalen Femurschaft, ausgebildet ist und einen kurzen Arm aufweist, und weiter vorgesehen ist
ein erster Metallblock mit parallelen Bohrungen an den medialen und lateralen Rändern und einem Fenster, in welches der kurze Arm abnehmbar aufgenommen ist, wobei der erste Metallblock nicht einstückig mit dem Träger ausgeführt ist, und
ein zweiter Metallblock, der an derselben Stelle wie der erste Metallblock anordenbar ist und Bohrungen aufweist, die an denselben geometrischen Positionen wie bei dem ersten Metallblock angeordnet sind, wobei der zweite Metallblock eine Schlittenführung für den Schneidblock aufweist.

2. System nach Anspruch 1, **dadurch gekennzeichnet, dass** es zusätzlich den Schneidblock umfasst.

3. System nach Anspruch 2,
**dadurch gekennzeichnet, dass**
der Schneidblock eine Plattform zur Aufnahme von medialen und lateralen Distanzstücken zur Winkelkorrektur mittels Oberflächenkontakt mit den Condylen des Femurs aufweist.

4. System nach Anspruch 3,
**dadurch gekennzeichnet, dass** es weiterhin Distanzstücke für die Plattform des Schneidblocks aufweist, wobei die Distanzstücke in Form von Metallstäben unterschiedlicher Dicke oder in Form einer Regelung mit Mikrometerskala mittels Kolbenbewegung ausgestaltet sind.

5. System nach einem der vorangehenden Ansprüche,
**dadurch gekennzeichnet, dass**
die Schlittenführung als Schwalbenschwanzführung ausgestaltet ist.

6. System nach einem der vorangehenden Ansprüche,
**dadurch gekennzeichnet, dass**
als Verbindung zwischen zweitem Metallblock und Schneidblock eine Gelenkverbindung vorgesehen ist.

## Claims

1. System for orientating a cutting block with a cutting slit on a bone, in particular on the distal end of the femur, comprising an L-shaped support, **characterized in that** the support is designed to be placed on the bone, in particular on the distal femoral shaft, and has a short arm, and there are moreover provided a first metal block, with parallel bores on the medial and lateral edges and with a slot in which the short arm is received in a removable manner, the first metal block not being designed in one piece with the support, and a second metal block, which can be arranged at the same location as the first metal block and has bores which are arranged at the same geometric positions as in the first metal block, wherein the second metal block has a slide guide for the cutting block.

2. System according to Claim 1, **characterized in that** it additionally comprises the cutting block.

3. System according to Claim 2, **characterized in that** the cutting block has a platform for receiving medial and lateral spacers for angle correction by means of surface contact with the condyles of the femur.

4. System according to Claim 3, **characterized in that** it moreover has spacers for the platform of the cutting block, wherein the spacers are configured in the form of metal rods of different thickness or in the form of a control with micrometre scale by means of piston movement.

5. System according to one of the preceding claims, **characterized in that** the slide guide is designed as a dovetail guide.

6. System according to one of the preceding claims, **characterized in that** a hinge connection is provided as connection between second metal block and cutting block.

## Revendications

1. Système pour l'orientation d'un bloc de coupe avec une fente de coupe sur un os, en particulier sur l'extrémité distale du fémur, comprenant un support en forme de L,
**caractérisé en ce que**
le support est configuré de façon à s'appliquer sur l'os, en particulier sur la diaphyse fémorale distale et présente un bras court, et
il est en outre prévu un premier bloc métallique avec des trous parallèles sur les bords médial et latéral et avec une fenêtre, dans laquelle le bras court est logé de façon amovible, dans lequel le premier bloc métallique n'est pas réalisé d'une seule pièce avec le support, et
un deuxième bloc métallique, qui peut être disposé au même endroit que le premier bloc métallique et qui présente des trous, qui sont disposés aux mêmes positions géométriques que dans le premier bloc métallique, dans lequel le deuxième bloc métallique présente un guidage de chariot pour le bloc de coupe.

2. Système selon la revendication 1, **caractérisé en ce qu'**il comprend en outre le bloc de coupe.

3. Système selon la revendication 2, **caractérisé en ce que** le bloc de coupe présente une plate-forme destinée à recevoir des pièces d'écartement médiale et latérale pour la correction angulaire au moyen d'un contact superficiel avec les condyles du fémur.

4. Système selon la revendication 3, **caractérisé en ce qu'**il présente en outre des pièces d'écartement pour la plate-forme du bloc de coupe, dans lequel les pièces d'écartement sont réalisées sous la forme de barres métalliques d'épaisseur différente ou sous la forme d'un réglage avec une échelle micrométrique au moyen du déplacement d'un piston.

5. Système selon l'une quelconque des revendications précédentes, **caractérisé en ce que** le guidage de chariot est réalisé sous la forme d'un guidage à queue d'aronde.

6. Système selon l'une quelconque des revendications précédentes, **caractérisé en ce qu'**il est prévu une liaison articulée comme liaison entre le deuxième bloc métallique et le bloc de coupe.
